# EUROPEAN PATENT APPLICATION

(11) **EP 3 047 846 A1**
(43) Date of publication of application: **27.07.2016**
(21) Application number: 15152170.5
(22) Date of filing: 22.01.2015
(51) Int. Cl.: A61K 9/10, A61K 47/36, A61K 31/00, A61P 11/04

(54) **DOSAGE FORMS CONTAINING FLUTICASONE PROPIONATE FOR THE TREATMENT OF INFLAMMATORY CONDITIONS OF THE ESOPHAGUS**

(71) Applicant: EMS S.A., 13186-901 Sao Paulo - SP (BR)
(72) Inventor: Santus, Giancarlo, Milano (IT); Khater Covesi, Leticia, Sao Paulo (BR); Donadoni, Luca, ALZANO LOMBARDO (IT); Ecclissato, Christina, 13186-901Sao Paulo (BR); Amazonas, Roberto, 13186-901Sao Paulo (BR)
(74) Representative: Cabinet Plasseraud

(57) **Abstract**

The present invention describes novel and improved dosage forms containing fluticasone propionate for the treatment of conditions associated with inflammation of the esophagus.

## Description

### Field of the invention

This invention relates to improved dosage forms of a steroid, for the treatment of inflammatory conditions of the esophagus. In particular the present invention relates to pharmaceutical compositions of fluticasone propionate with good bioavailability, reduced side effects, enhanced patient compliance, and a method for obtaining said improved dosage forms for the treatment of eosinophilic esophagitis.

### Background

Eosinophilic esophagitis is an allergic inflammatory disease characterized by elevated eosinophils in the esophagus. This inflammatory condition of the esophagus affects both children and adults, and men more than women.

Eosinophilic esophagitis is a newly recognized disease that over the past decade has been increasingly diagnosed. It is a rare disease, but increasing in prevalence to an estimated 1:2000. This increase is thought to reflect an increase in diagnosis, as well as a true increase in eosinophilic esophagitis cases. Fortunately, the medical community is responding and new scientific information is emerging to guide management of this disorder, which often persists with ongoing or recurrent symptoms.

The major symptom in adults with eosinophilic esophagitis is dysphagia (problems of swallowing) for solid food. Eosinophilic esophagitis stiffens the esophagus so that solid foods have difficulty passing through the esophagus into the stomach. Other common causes of dysphagia for solid food are esophageal strictures and Schatzki rings. The diagnosis of eosinophilic esophagitis is usually made during an endoscopy (EGD), performed for the evaluation of dysphagia and is confirmed by biopsy of the esophagus.

People with eosinophilic esophagitis commonly have other allergic diseases such as rhinitis, asthma, and/or eczema. Eosinophilic esophagitis can be driven by food allergy or intolerance: most patients who eliminate food proteins from their diet (by drinking only an amino-acid based formula) improve their condition. The disease may also be triggered by other environmental factors that researchers are beginning to understand.

There are a number of studies that have demonstrated the central role of food allergens in triggering eosinophilic esophagitis. When these allergenic foods are removed from a person's diet, symptoms can resolve and the eosinophilic inflammation in the esophagus can be healed.

In addition, there are medical therapies available - the most common drug therapy is the use of swallowed inhaled steroids such as budesonide (Pulmicort®) made into a slurry or fluticasone (Flovent®) inhalers. Presently these therapies are off-label treatments as there are no approved drug medications with this clinical indication.

The selected steroid of this invention is fluticasone propionate, a compound with potent anti-inflammatory activity widely used for the treatment of respiratory diseases.

Fluticasone propionate is an old drug firstly described in British Patent GB2088877. There are several different dosage forms commercially available, such as oral tablets, inhalation dry powders, oral inhalation aerosol, nasal sprays, topical dermatological cream and ointments.

Novel fluticasone formulations with at least one surface stabilizer and fluticasone particles of an average size of less than about 2000 nm are described in WO2004/069225. The formulations described in this patent application are mainly for treating respiratory related illness. There is no mention of gastrointestinal diseases in general nor of eosinophilic esophagitis specifically.

Orally administered corticosteroid compositions are described in WO2011/041509. This patent application is primarily directed to solid dispersible dosage forms also in presence of bioadhesive polymers. Moreover, liquid bioadhesive formulations are described, mainly non aqueous. There is no mention of viscous sugar free aqueous liquid formulations of fluticasone propionate.

None of the known prior art describes a viscous sugar free liquid formulation of fluticasone propionate specifically designed for the treatment of eosinophilic esophagitis.

There is therefore a need in the art for orally administered steroid formulations specifically designed for the treatment of eosinophilic esophagitis that can decrease frequency of administration, improve clinical efficacy and reduce side effects associated with the use of steroids.

### Summary of the invention

An embodiment of the present invention is a liquid composition comprising fluticasone propionate.

In another embodiment the present invention is directed to a sugar free liquid fluticasone propionate formulation.

In another embodiment the formulation is a high viscosity formulation to enhance the contact time with the esophageal mucosa.

In another embodiment the invention is directed to a liquid viscous composition comprising sodium alginate.

In another embodiment the invention is directed to a viscous liquid dosage form comprising fluticasone propionate in combination with sodium alginate and other non-active ingredients. The viscous liquid dosage form thus comprises fluticasone propionate, sodium alginate and other non-active ingredients.

In another embodiment, the present invention is directed to a process for the preparation of a dosage form containing fluticasone propionate and a thickening agent, preferably sodium alginate.

In yet another embodiment the present invention is directed to a method of treating eosinophilic esophagitis.

### Description of the invention

The present invention provides pharmaceutical compositions of fluticasone propionate for oral administration wherein said compositions provide a better patient treatment with good bioavailability, good tolerability and an improved compliance thanks to the increase of viscosity of the compositions and to the reduction of number of administrations.

The invention is thus directed to a viscous liquid dosage form comprising fluticasone propionate in combination with sodium alginate and other non-active ingredients. In particular, the present invention deals with formulations comprising fluticasone propionate and sodium alginate in the form of viscous sugar free syrup. Thus, preferably, the viscous liquid dosage form is a sugar free syrup.
The invention is also directed to a liquid pharmaceutical composition comprising fluticasone propionate in combination with sodium alginate and other non-active ingredients.

The fluticasone propionate in the composition may have a D₉₀ of less than 10 microns; D₉₀ being defined as the diameter where 90% of the particles have a smaller particle size and 10% a bigger one.

The fluticasone propionate of the invention can be in a crystalline or amorphous form, as well as a mixture thereof.

The amount of fluticasone propionate in the present invention will be selected so as to maximize the therapeutic benefit depending on the status and age of the patient and severity of the disease. Preferably it will be in the range 0.01 mg to 20 mg/ dose.
Preferably, fluticasone propionate is suspended in the viscous liquid dosage form, preferably in the syrup formulation.

The present invention, in at least one of the aforementioned aspects, can show one or more of the preferred characteristics described below.

The other non-active ingredients of the dosage form or composition of the invention are preferably chosen from edulcorants, suspending agents, surfactants and preservatives. The other non-active ingredients of the dosage form or composition of the invention preferably comprise edulcorants, suspending agents, surfactants and preservatives.

The dosage form or composition of the invention preferably comprises a surfactant. Suitable surfactants include sodium lauryl sulfate, sodium docusate, cetylpyridinium chloride, lecithin, poloxamers (such as those marketed under the name Pluronic), monoglycerides, sorbitan esters of fatty acids (such as those marketed under the name Span) and polysorbates (or polyethoxylated sorbitan esters of fatty acids, such as those marketed under the name Tween). Preferably, the surfactant is selected from poloxamers, monoglycerides, sorbitan esters of fatty acids and polysorbates.

Typically, the surfactant used in the dosage form or composition of the present invention is polysorbate 80 or polysorbate 40.

Preferably, the dosage form or composition of the present invention includes an amount of surfactant, preferably polysorbate, between 0.001% and 0.01% w/w relative to the total weight of said pharmaceutical dosage form or composition.

The dosage form or composition of the invention preferably comprises a thickening agent to increase viscosity. Suitable thickening agents include xantan gum, pectins, chitosan derivatives, carrageenans, guar gum, cellulose ethers, povidone, alginic acid and sodium alginate.

Typically, the thickening agent used in the formulation of the present invention is sodium alginate. Preferably, the dosage form or composition includes an amount of sodium alginate between 0.5% and 10% w/w, depending of the viscosity of the polymer used. Preferably, the dosage form or composition includes a sodium alginate with a viscosity of 50-1500 mPa.s in 1% aqueous solution.

The dosage form or composition of the invention preferably comprises also a suspending agent. Suitable suspending agents include cellulose ethers, microcrystalline cellulose and its derivatives, magnesium aluminium silicates (such as those marketed under the name Veegum) and carbomers. Preferably, the suspending agent is chosen from cellulose ethers, magnesium aluminium silicates and carbomers.

Typically, the suspending agent used in the dosage form or composition of the present invention is a carbomer. Preferably, the dosage form or composition includes an amount of suspending agent, preferably carbomer, between 0.1% and 0.5% w/w relative to the total weight of said pharmaceutical dosage form or composition.

The dosage form or composition of the invention preferably comprises a sweetener agent, also called edulcorant in the present invention. Suitable sweetener agents (edulcorants) include aspartame, cyclamates such as sodium cyclamate or calcium cyclamate, acesulfame potassium, sucralose, saccharin sodium, xylitol and sorbitol.

Typically, the edulcorant used in the dosage form or composition of the present invention is saccharin sodium. Preferably, the dosage form or composition includes an amount of edulcorant, preferably saccharin sodium, between 0.05% and 0.5% w/w relative to the total weight of said pharmaceutical dosage form or composition.

The dosage form or composition of the invention preferably comprises a preservative to prevent microbial contamination. Suitable preservatives include chlorobutanol, benzyl alcohol, boric acid, sorbic acid and their respective salts, such as potassium sorbate, glycerin, propylene glycol, phenol, chlorocresol, O-phenyl phenol, benzoic acid, and alkyl esters of parahydroxybenzoic acid, such as methylparaben or propylparaben, or mixtures thereof. Preferably, the preservative is selected from potassium sorbate, benzoic acid, methylparaben, propylparaben and mixtures thereof.

Typically, the preservative used in the dosage form or composition of the present invention is a mixture of methyl parahydroxybenzoate (methylparaben) and propyl parahydroxybenzoate (propylparaben).

Preferably, the dosage form or composition includes an amount of preservative, preferably methyl parahydroxybenzoate, between 0.01% and 0.5% w/w, preferably between 0.05% and 0.5% w/w, more preferably between 0.15 and 0.4%. Preferably, the dosage forms or compositions include an amount of methyl parahydroxybenzoate between 0.05% and 0.5% w/w, more preferably between 0.15 and 0.4%, and an amount of propyl parahydroxybenzoate between 0.01% and 0.1% w/w relative to the total weight of said pharmaceutical dosage form or composition.

The dosage form or composition of the invention preferably comprises a solid or liquid flavor, such as volatile oils (e.g., orange oil), vanillin, and others, to render the syrup with a pleasant tasting. These flavors must possess sufficient water-solubility. Typical flavoring agents which are commonly used in sweetened pharmaceuticals, foods, candies or beverages, are also useful in the present invention. These materials may impart flavors such as flavor of red fruits, green apple, grape, cherry, citrus, peach, strawberry, bubble gum, peppermint and many others and are within the scope of the present invention. Preferred flavoring agents are Flavor Red Fruits and Blood Orange.

Preferably, the dosage form or composition includes an amount of flavour between 0.1% and 1 % w/w.

The dosage form or composition of the present invention can moreover comprise other pharmaceutically acceptable additives conventionally known by a person skilled in the art, such as for example, antioxidants, buffering agents, chelating agents and colorants.

Useful examples of antioxidants are BHA, BHT, malic acid, ascorbic acid, alpha tocopherol and propyl gallate at the concentration of 0.01-0.1% w/w.

Useful examples of buffering agents are citric acid and citrates of sodium or potassium, phosphoric acid and phosphates of sodium and of potassium. The combination of citric acid and sodium citrate is particularly preferred. The amount of buffering agent needed is generally between 0.01 and 0.1 M, and a concentration between 0.05 and 0.5 M is usually sufficient.

In a preferred embodiment, the pH of the dosage form or composition is between about 6 and about 8. Preferably, the pH is between about 6.5 and about 7.5, more preferably the pH is about 7.

The present invention also relates to a process for the preparation of a dosage form containing fluticasone propionate and a thickening agent, said process comprising the steps of:
(i) suspending the fluticasone propionate in water with a surfactant;
(ii) adding suspension (i) to a preservative solution;
(iii) dispersing a suspending agent and neutralizing;
(iv) adding phase (ii) to phase (iii) and adding a thickening agent; and
(v) adding flavour, adjusting pH and filling into bottles.
All the above described features are applicable to the process.

The invention also relates to a dosage form as described above for use for the treatment or prophylaxis of eosinophilic esophagitis. Preferably, this treatment is administered to a subject in an effective amount, which is preferably in the range of 0.01 mg to 20 mg per dose.

The present invention will now be illustrated by the following examples. It is understood, however, that such examples are provided for illustration only, and the invention is not intended to be limited by the examples. The pharmaceutical compositions based on the system employed in the examples can be formed by any suitable method known in the art.

### Example 1. Pharmaceutical composition of fluticasone propionate sugar free syrup

| **Composition** | **%** |
|---|---|
| Fluticasone propionate | 0.01 |
| Sodium alginate | 2.0 |
| Carbomer | 0.1 |
| Saccharin sodium | 0.2 |
| Methyl parahydroxybenzoate | 0.4 |
| Propyl parahydroxybenzoate | 0.06 |
| Blood orange flavour | 0.2 |
| Polysorbate 80 | 0.002 |
| Sodium hydroxide 1M | q.s. |
| Purified water | q.s.to 100 |

### Preparation process of the suspension

1. Disperse the carbomer into purified water until complete hydratation of the polymer. Neutralize with 1 M Sodium hydroxide (gel formation).
2. Solubilize the propyl parahydroxybenzoate and methyl parahydroxybenzoate into boiling purified water. Add the saccharin sodium.
3. Disperse the fluticasone propionate into purified water at room temperature and add the polysorbate 80 to facilitate the process.
4. Add the API (Active Pharmaceutical Ingredient) suspension to the preservative solution.
5. Disperse the phase 4 into the phase 1, add the sodium alginate and keep stirring for 40 minutes (mechanical stirring).
6. Add the flavour, bring to pH 6.5-7.5 with sodium hydroxide 1M and add purified water up to final weight.

### Example 2. Pharmaceutical composition of fluticasone propionate sugar free syrup

| **Composition** | **%** |
|---|---|
| Fluticasone propionate | 0.01 |
| Sodium alginate | 3.0 |
| Carbomer | 0.5 |
| Saccharin sodium | 0.2 |
| Methyl parahydroxybenzoate | 0.15 |
| Propyl parahydroxybenzoate | 0.05 |
| Blood orange flavour | 0.2 |
| Polysorbate 80 | 0.002 |
| Sodium hydroxide 1M | q.s. |
| Purified water | q.s.to 100 |

The preparation process is the same of example 1.

### Example 3. Pharmaceutical composition of fluticasone propionate sugar free syrup

| **Composition** | **%** |
|---|---|
| Fluticasone propionate | 0.01 |
| Sodium alginate | 10.0 |
| Saccharin sodium | 0.2 |
| Methyl parahydroxybenzoate | 0.15 |
| Propyl parahydroxybenzoate | 0.05 |
| Mint flavour | 0.2 |
| Polysorbate 80 | 0.002 |
| Sodium hydroxide 1M | q.s. |
| Purified water | q.s. to 100 |

The preparation process is the same of example 1.

### Example 4. Pharmaceutical composition of fluticasone propionate sugar free syrup

| **Composition** | **%** |
|---|---|
| Fluticasone propionate | 0.01 |
| Sodium alginate | 1.0 |
| Cellulose microcrystalline and carboxymethylcellulose sodium | 0.5 |
| Saccharin sodium | 0.2 |
| Methyl parahydroxybenzoate | 0.15 |
| Propyl parahydroxybenzoate | 0.05 |
| Blood orange flavour | 0.2 |
| Polysorbate 80 | 0.002 |
| Sodium hydroxide 1M | q.s. |
| Purified water | q.s. |

### Preparation process

1. Disperse cellulose microcrystalline and carboxymethylcellulose sodium into purified water until complete hydratation of the polymer.
2. Solubilize the propyl parahydroxybenzoate and methyl parahydroxybenzoate into boiling purified water. Add the saccharin sodium.
3. Disperse the fluticasone propionate into purified water at room temperature and add the polysorbate 80 to facilitate the process.
4. Add the API suspension to the preservative solution.
5. Disperse the phase 4 into the phase 1, add the sodium alginate and keep stirring.
6. Add the flavour, bring to pH 6.5-7.5 with sodium hydroxide 1M and add purified water up to final weight.

### Example 5. Viscosity of the free sugar syrup

Preparations containing alginates show a typical thixotropic behavior. So, it is important to perform the analysis with constant conditions.

Formulations of previous examples show viscosity values between 500 and 5000 mPa·s, depending on the type of sodium alginate used. All the analyses were performed with a Brookfield LVT viscometer at 24 ±0.5 °C, with spindle number 2, at 1.5 RPM.

### Example 6. Stability

The stability of the formulations prepared as described in Examples 1 to 4 was checked for assay, related substances and viscosity after 6 months at room temperature and in accelerated conditions, according to ICH guidelines, and there were no significant changes from initial results.

Microbial testing was also conducted on samples at the beginning of the stability study and after 6 months at 30°C/65% RH (Room Humidity). The microbial quality was found to be satisfactory. That is, to have a total aerobic microbial count of no more than 100 bacteria/mL, total molds and yeast count of no more than 10 fungi/mL, and absence of E. coll, P. aeruginosa, S aureus, and Salmonella sp.

### Example 7. Adhesion

We tested *in vitro* the adhesion properties of our high viscosity formulation versus a regular fluticasone propionate suspension. We compared the formulation of Example 1, with Flixonase® nasal spray suspension 0.05% (batch n° 1027400051). Oral use of steroidal suspensions in the form of MDI (meter dose inhaler), DPI (dry powder inhaler), nasal spray, such as fluticasone propionate (Flixonase®), is suggested as present therapy of eosinophilic esophagitis.

An amount of about 50 mg of both preparations was positioned on a vertical glass surface and the time that the two preparations spend to cover 5 cm at room temperature was measured.

Flixonase suspension spends 5 seconds to cover the distance, while our viscous formulation spends more than 10 minutes.

The results obtained with this simple test suggest that the viscous formulation of the present invention may represent, with its prolonged contact time, an improved alternative to current therapies for eosinophilic esophagitis.

## Claims

1. A viscous liquid dosage form comprising fluticasone propionate in combination with sodium alginate and other non-active ingredients.

2. Dosage form according to claim 1, wherein it is a sugar free syrup.

3. Dosage form according to claim 1 or 2, wherein said non-active ingredients comprise edulcorant(s), suspending agent(s), surfactant(s) and preservative(s).

4. Dosage form according to claim 3, wherein said edulcorant is selected from aspartame, saccharin sodium, acesulfame potassium, sucralose, cyclamates, xylitol and sorbitol.

5. Dosage form according to claim 3 or 4, comprising between 0.05% and 0.5% w/w of said edulcorant.

6. Dosage form according to claim 3, wherein said suspending agent is selected from cellulose ethers, carbomers and aluminium magnesium silicates.

7. Dosage form according to claim 3 or 6, comprising between 0.1% and 0.5% w/w of said suspending agent.

8. Dosage form according to claim 3, wherein said surfactant is selected from poloxamers, monoglycerides, sorbitan esters of fatty acids and polysorbates.

9. Dosage form according to claim 3 or 8 comprising between 0.001% and 0.01% w/w of said surfactant.

10. Dosage form according to claim 3 wherein said preservative is selected from methylparaben, propylparaben, potassium sorbate and benzoic acid.

11. Dosage form according to claim 3 or 10 comprising between 0,01% and 0,5% w/w of said preservative.

12. Dosage form according to any one of claims 1 to 11, wherein fluticasone propionate is suspended in the viscous liquid dosage form, preferably in a syrup formulation.

13. A process for the preparation of a dosage form containing fluticasone propionate and a thickening agent, said process comprising the steps of:
(i) suspending the fluticasone propionate in water with a surfactant;
(ii) adding suspension (i) to a preservative solution;
(iii)dispersing a suspending agent and neutralizing;
(iv)adding phase (ii) to phase (iii) and adding a thickening agent;
(v) adding flavour, adjusting pH and filling into bottles.

14. The process according to claim 13 wherein the pH of said dosage form is between 6 and 8, more preferably between 6.5 and 7.5.

15. The process according to claim 13 or 14 wherein said surfactant is polysorbate between 0.001% and 0.01% w/w.

16. The process according to any one of claims 13 to 15 wherein said preservative solution comprises parahydroxybenzoate between 0.05% and 0.5% w/w and propyl parahydroxybenzoate between 0.01% and 0.1% w/w.

17. The process according to any one of claims 13 to 16 wherein said suspending agent is carbomer, present in an amount between 0.1% and 0.5% w/w.

18. The process according to any one of claims 13 to 17 wherein the thickening agent is sodium alginate with a viscosity of 50-1500 mPa.s in 1% aqueous solution.

19. A liquid pharmaceutical composition comprising fluticasone propionate in combination with sodium alginate and other non-active ingredients.

20. The liquid pharmaceutical composition according to claim 19 wherein said non-active ingredients comprise edulcorants, suspending agents, surfactants and preservatives.

21. The liquid pharmaceutical composition according to claim 20, wherein said edulcorant(s) is selected from aspartame, sodium saccharin, acesulfame, cyclamate, xylitol and sorbitol.

22. The liquid pharmaceutical composition according to claim 20, wherein said suspending agent(s) is selected from cellulose ethers, carbomers and aluminium magnesium silicates.

23. The liquid pharmaceutical composition according to claim 20, wherein said surfactant is selected from poloxamers, monoglycerides, sorbitan esters of fatty acids and polysorbates.

24. The liquid pharmaceutical composition according to claim 20, wherein said preservative is selected from methylparaben, propylparaben, potassium sorbate and benzoic acid.

25. A dosage form according to any one of claims 1 to 12, for use for the treatment or prophylaxis of eosinophilic esophagitis.
